# EUROPEAN PATENT APPLICATION

(11) **EP 4 837 896 A1**
(43) Date of publication of application: **11.11.2026**
(21) Application number: 24914914.7
(22) Date of filing: 27.09.2024
(51) Int. Cl.: C07C 7/10, C07C 7/08, C07C 15/02

(54) **METHOD FOR SEPARATING VINYLTOLUENE AND SATURATED C9 AROMATIC HYDROCARBON, AND EXTRACTANT MIXTURE**

(30) Priority: 02.01.2024 CN 202410007868
(71) Applicant: China Petroleum & Chemical Corporation, Beijing 100728 (CN); Sinopec (Beijing) Research Institute of Chemical Industry Co., Ltd., Beijing 100013 (CN)
(72) Inventor: CUI, Ting, Beijing 100013 (CN); WANG, Yufei, Beijing 100013 (CN); CUI, Zhonghui, Beijing 100013 (CN); WANG, Jing, Beijing 100013 (CN); GUO, Liang, Beijing 100013 (CN); YANG, Bo, Beijing 100013 (CN); CHANG, Dashan, Beijing 100013 (CN); CHEN, Jian, Beijing 100013 (CN); LIU, Jiannan, Beijing 100013 (CN); SONG, Xiaoqian, Beijing 100013 (CN); SUN, Song, Beijing 100013 (CN); ZHANG, Quanzhen, Beijing 100013 (CN)
(74) Representative: karo IP
(86) International application number: PCT/CN2024/121621
(87) International publication number: WO 2025/145693

(57) **Abstract**

The present disclosure relates to a method and an extractant mixture for separating vinyltoluene and C9 saturated aromatic hydrocarbons represented by C₉H₁₂. The method in accordance with the present disclosure comprises steps of: (1) providing a stream containing vinyltoluene and C9 saturated aromatic hydrocarbons; (2) feeding the stream and an extractant mixture into an extractive distillation column and subjecting them to extractive distillation, to obtain a top fraction and a bottom fraction, wherein the extractant mixture comprises N,N-dimethylformamide and glycerol, the top fraction comprises C9 saturated aromatic hydrocarbons and N,N-dimethylformamide, and the bottom fraction comprises vinyltoluene and glycerol. The method in accordance with the present disclosure can be used to separate vinyltoluene and C9 saturated aromatic hydrocarbons from light cracked C9 aromatic hydrocarbons, with high yield and high purity.

## Description

### Technical Field

The present disclosure relates to separation of aromatic hydrocarbons, particularly to a method and an extractant mixture for separating vinyltoluene and C9 saturated aromatic hydrocarbons from light cracked C9 aromatic hydrocarbons.

### Background

C9 cracking fraction is one of by-products of petroleum cracking, containing a large amount of C9 aromatic hydrocarbons (also known as cracked C9 aromatic hydrocarbons), such as indene, vinyltoluene, propylbenzene, methylethylbenzene, trimethylbenzene and the like. Currently, in most cases, cracked C9 aromatic hydrocarbons are used directly without treatment, for example, as a gasoline blending component. In some cases, they are also used to produce petroleum resins with very low added value, wherein most components contained therein are not fully utilized. Direct use of cracked C9 aromatic hydrocarbons may lead to low utilization of resource and low economic added value.

Many methods have been proposed in the art to separate one or some components from C9 cracking fraction or cracked C9 aromatic hydrocarbons. For example, CN107502386A discloses a method for obtaining mixed aromatic hydrocarbons by hydrogenation and separation of C9 cracking fraction. The method includes: removing heavy components such as colloids from the C9 cracking fraction; subjecting to low-temperature liquid phase hydrogenation to remove easily polymerizable components such as diolefins; subjecting to high-temperature gas phase hydrogenation to remove monoolefins, sulfur and nitrogen; and subjecting to rectification separation to obtain mixed aromatic hydrocarbons with low non-aromatic content. The hydrogenations therein also remove active monomers such as styrene in the C9 cracking fraction, thereby resulting in low utilization of resource, high cost and low economic added value.

Vinyltoluene, such as 2-methylstyrene, is an important monomer for organic synthesis, especially in pharmaceutical research and development. Vinyltoluene is synthesized in the art by reacting ethanol with toluene. Such synthesis involves disadvantages, such as expensive raw materials, low reaction efficiency, high energy consumption, high equipment investment, and the like. Vinyltoluene is involved in cracked C9 aromatic hydrocarbons. Therefore, efficient separation of vinyltoluene from cracked C9 aromatic hydrocarbons will be a favorable alternative to the above synthesis.

Components in cracked C9 aromatic hydrocarbons may have closed boiling points. Among them, 2-methylstyrene has a boiling point of about 169°C, 3-methylstyrene has a boiling point of about 170°C, and 4-methylstyrene has a boiling point of about 171°C. Among the alkylsubstituted benzenes with nine carbon atoms (C9 saturated aromatic hydrocarbons), 2-ethyltoluene has a boiling point of about 165°C, 4-ethyltoluene has a boiling point of about 162°C, mesitylene has a boiling point of about 167°C, and para-trimethylbenzene has a boiling point of about 168°C. Since vinyltoluene and C9 saturated aromatic hydrocarbons have closed boiling points and similar structures, it is difficult to separate them from each other using ordinary rectification.

Extractive distillation has been proposed in the art to separate materials. However, there is no report on the use of extractive distillation to separate vinyltoluene and C9 saturated aromatic hydrocarbons with boiling points close to that of vinyltoluene.

### Summary of the invention

The present disclosure is to overcome the problem that it is difficult to separate vinyltoluene and C9 saturated aromatic hydrocarbons in the prior art. Provided in the present disclosure is a method for separating vinyltoluene and C9 saturated aromatic hydrocarbons from a stream containing vinyltoluene and C9 saturated aromatic hydrocarbons, wherein the separating is operated via extractive distillation by using an extractant mixture containing N,N-dimethylformamide and glycerol. The method in accordance with the present disclosure can be used to separate vinyltoluene and C9 saturated aromatic hydrocarbons from (light) cracked C9 aromatic hydrocarbons, with high yield and high purity.

In one aspect, provided in the present disclosure is a method for separating vinyltoluene and C9 saturated aromatic hydrocarbons represented by C₉H₁₂, comprising steps of:
(1) providing a stream containing vinyltoluene and C9 saturated aromatic hydrocarbons;
(2) feeding the stream and an extractant mixture into an extractive distillation column and subjecting them to extractive distillation, to obtain a top fraction and a bottom fraction, wherein the extractant mixture comprises a mixed solution of N,N-dimethylformamide and glycerol, the top fraction comprises C9 saturated aromatic hydrocarbons and N,N-dimethylformamide, and the bottom fraction comprises vinyltoluene and glycerol.

In another aspect, provided in the present disclosure is a method for separating vinyltoluene and C9 saturated aromatic hydrocarbons represented by C₉H₁₂ from light cracked C9 aromatic hydrocarbons, comprising steps of:
(1) feeding light cracked C9 aromatic hydrocarbons and an extractant mixture into an extractive distillation column and subjecting them to extractive distillation, to obtain a top fraction and a bottom fraction, wherein the extractant mixture comprises N,N-dimethylformamide and glycerol, the top fraction comprises C9 saturated aromatic hydrocarbons and N,N-dimethylformamide, and the bottom fraction comprises vinyltoluene and glycerol.

In a further aspect, provided in the present disclosure is an extractant mixture for separating vinyltoluene and C9 saturated aromatic hydrocarbons represented by C₉H₁₂ via extractive distillation, wherein the extractant mixture comprises N,N-dimethylformamide and glycerol.

The inventors of the present invention surprisingly found that vinyltoluene and C9 saturated aromatic hydrocarbons can be effectively separated via extractive distillation by using an extractant mixture comprising N,N-dimethylformamide (DMF) and glycerol (GLY). The present invention was completed based on the above finding. The method in accordance with the present disclosure can continuously separate vinyltoluene and C9 saturated aromatic hydrocarbons from light cracked C9 aromatic hydrocarbons, with high yield and high purity. For example, purity of vinyltoluene may be greater than or equal to 90%, and yield thereof may be greater than or equal to 85%; and purity of C9 saturated aromatic hydrocarbons may be greater than or equal to 65%.

### DESCRIPTION OF THE DRAWINGS

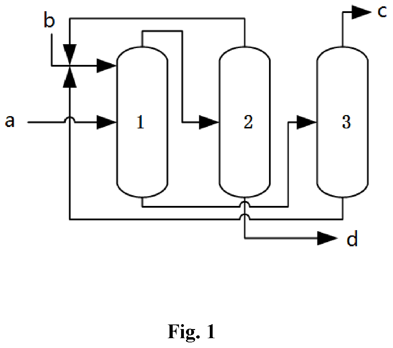

Fig. 1 is a process flow diagram of one embodiment of the method in accordance with the present disclosure.
reference signs
1: an extractive distillation column;
2: a refining column for C9 saturated aromatic hydrocarbons;
3: a refining column for vinyltoluene;
a: light cracked C9 aromatic hydrocarbons;
b: an extractant mixture;
c: a stream enriched in C9 saturated aromatic hydrocarbons;
d: a stream enriched in vinyltoluene.

### Detailed description

The embodiments of the invention will be further described hereinafter in detail. It should be noted that the described embodiments are provided for illustration purpose only, and are not intended to be limiting in any manner.

It should be understood that any values disclosed herein (including the endpoints in the ranges) are not limited to the precise value, but to encompass values close to those values, for example, all possible values within a range of the precise value ± 5%. In addition, for ranges of values, it is possible to combine between the endpoints of each of the ranges, between the endpoints of each of the ranges and the individual points, and between the individual points to give one or more new ranges of values as if these new ranges of values are specifically disclosed herein.

Other than in the examples, all values of parameters in this specification are to be understood as being modified in all instances by the term "about" whether or not "about" actually appears before the values.

Unless otherwise stated, throughout the specification and claims, the term "comprise" or its variations such as "comprises" or "comprising" and the like shall be understood to include the stated parts or components, without excluding other parts or other components. In addition, the term "comprise" or its variations such as "comprises" or "comprising" may also exclude other parts or other components, that is, the situation of "consisting of..." or "consisting essentially of...".

In one aspect, provided in the present disclosure is a method for separating vinyltoluene and C9 saturated aromatic hydrocarbons represented by C₉H₁₂, comprising steps of:
(1) providing a stream containing vinyltoluene and C9 saturated aromatic hydrocarbons;
(2) feeding the stream and an extractant mixture into an extractive distillation column and subjecting them to extractive distillation, to obtain a top fraction and a bottom fraction, wherein the extractant mixture comprises N,N-dimethylformamide and glycerol, the top fraction comprises C9 saturated aromatic hydrocarbons and N,N-dimethylformamide, and the bottom fraction comprises vinyltoluene and glycerol.

As used herein, "vinyltoluene" is selected from 1-methylstyrene, 2-methylstyrene, 3-methylstyrene and mixtures thereof. The boiling point of vinyltoluene depends on the respective amounts of 1-methylstyrene, 2-methylstyrene and 3-methylstyrene, which is generally 169-171°C. In one variation, the vinyltoluene is 2-methylstyrene.

As used herein, "C9 saturated aromatic hydrocarbons represented by C₉H₁₂ " refers to a benzene ring with all substituents being alkyl groups which has nine carbon atoms, which may include one or more of propylbenzene, methylethylbenzene and trimethylbenzene. Some components of C9 saturated aromatic hydrocarbons, such as 2-ethyltoluene (having a boiling point of about 165°C), 4-ethyltoluene (having a boiling point of about 162°C), mesitylene (having a boiling point of about 167°C), para-trimethylbenzene (having a boiling point of about 168°C), and the like, have the same or similar boiling points as that of the vinyltoluene.

In one variant, the stream containing vinyltoluene and C9 saturated aromatic hydrocarbons consists of vinyltoluene and C9 saturated aromatic hydrocarbons, or consists essentially of vinyltoluene and C9 saturated aromatic hydrocarbons. In the latter embodiment, vinyltoluene and C9 saturated aromatic hydrocarbons comprise equal to or greater than 85% by weight, preferably equal to or greater than 90% by weight, more preferably equal to or greater than 95% by weight, and most preferably equal to or greater than 98% of the stream.

The extractant mixture used in the invention comprises N,N-dimethylformamide and glycerol. In one embodiment, the extractant mixture used in the invention consists of N,N-dimethylformamide and glycerol. In another embodiment, the extractant mixture comprises N,N-dimethylformamide, glycerol and an auxiliary extractant, wherein the auxiliary extractant is selected from a group consisting of sulfolane, dimethyl sulfoxide and phenol. The auxiliary extractant is selected in an amount so that the extractive distillation is not affected. In a preferred embodiment, the extractant mixture comprises 0-50% by weight, preferably 0-20% by weight, and more preferably 0-10% by weight of the auxiliary extractant. N,N-dimethylformamide and glycerol are present in a total amount of 50-100% by weight, preferably 80-100% by weight, and more preferably 90-100% by weight of the extractant mixture. Preferably, in the above two embodiments, N,N-dimethylformamide and glycerol are present in a mass ratio of 0.05-0.5:1, preferably 0.06-0.12:1. The inventors of the present invention surprisingly found that controlling N,N-dimethylformamide and glycerol in a mass ratio within the above range is helpful for obtaining a better separation effect.

According to a preferred embodiment of the present invention, the top fraction and the bottom fraction may be refined separately, to obtain vinyltoluene and C9 saturated aromatic hydrocarbons. For example, the top fraction which comprises C9 saturated aromatic hydrocarbons and N,N-dimethylformamide may be refined in a refining column for C9 saturated aromatic hydrocarbons, to obtain N,N-dimethylformamide at the top of the refining column and a stream enriched in C9 saturated aromatic hydrocarbons at the bottom of the refining column. The bottom fraction which comprises vinyltoluene and glycerol may be refined in a refining column for vinyltoluene, to obtain a stream enriched in vinyltoluene at the top of the refining column and glycerol at the bottom of the refining column. In the case where the extractant mixture contains an auxiliary extractant, the bottom fraction may comprise vinyltoluene, glycerol and the auxiliary extractant. Accordingly, the bottom fraction may be refined in a refining column for vinyltoluene, to obtain a stream enriched in vinyltoluene at the top of the refining column and a stream comprising glycerol and the auxiliary extractant at the bottom of the refining column.

The method in accordance with the present disclosure can effectively separate vinyltoluene and C9 saturated aromatic hydrocarbons represented by C₉H₁₂, thereby being particularly suitable for separating vinyltoluene and C9 saturated aromatic hydrocarbons represented by C₉H₁₂ from light cracked C9 aromatic hydrocarbons. Accordingly, provided in the present disclosure is a method for separating vinyltoluene and C9 saturated aromatic hydrocarbons represented by C₉H₁₂ from light cracked C9 aromatic hydrocarbons, comprising a step of:
(1) feeding light cracked C9 aromatic hydrocarbons and an extractant mixture into an extractive distillation column and subjecting them to extractive distillation, to obtain a top fraction and a bottom fraction, wherein the extractant mixture comprises N,N-dimethylformamide and glycerol, the top fraction comprises C9 saturated aromatic hydrocarbons and N,N-dimethylformamide, and the bottom fraction comprises vinyltoluene and glycerol.

In a preferred embodiment, the method in accordance with the present disclosure further comprises steps of:
(2) feeding the top fraction of step (1) into a refining column for C9 saturated aromatic hydrocarbons and subjecting it to refining, to obtain N,N-dimethylformamide at the top of the refining column and a stream enriched in C9 saturated aromatic hydrocarbons at the bottom of the refining column;
(3) feeding the bottom fraction of step (1) into a refining column for vinyltoluene and subjecting it to refining, to obtain a stream enriched in vinyltoluene at the top of the refining column and glycerol at the bottom of the refining column.

As used herein, the term "light cracked C9 aromatic hydrocarbons" refers to a stream obtained after removing heavy components from cracked C9 aromatic hydrocarbons. The term "cracked C9 aromatic hydrocarbons" refers to one of the by-products of petroleum cracking, which comprises aromatic fractions with nine carbon atoms. The aromatic fractions may include indene, vinyltoluene, propylbenzene, methylethylbenzene and trimethylbenzene, and the like, which have different boiling points respectively. Cracked C9 aromatic hydrocarbons may further comprise fractions with more than nine carbon atoms, such as dicyclopentadiene. Generally, components having boiling points greater than or equal to the boiling point of indene (about 182°C) are regarded as heavy components of cracked C9 aromatic hydrocarbons. Light cracked C9 aromatic hydrocarbons are obtained after removing those heavy components from cracked C9 aromatic hydrocarbons, which may include vinyltoluene, propylbenzene, methylethylbenzene and trimethylbenzene. Light cracked C9 aromatic hydrocarbons may contain some impurities, such as residual heavy components. Obviously, light cracked C9 aromatic hydrocarbons are also a stream comprising vinyltoluene and C9 saturated aromatic hydrocarbons.

The removing of the heavy components from cracked C9 aromatic hydrocarbons may be operated using various methods known in the art. For example, cracked C9 aromatic hydrocarbons may be treated in two columns, wherein, in the first column, dicyclopentadiene in cracked C9 aromatic hydrocarbons is depolymerized into cyclopentadiene and cyclopentadiene is removed. Then, the stream obtained after removing dicyclopentadiene is fed into the second column for removing other heavy components, to obtain light cracked C9 aromatic hydrocarbons.

The extractant mixture used in the present invention comprises N,N-dimethylformamide and glycerol. In one embodiment, the extractant mixture used in the present invention consists of N,N-dimethylformamide and glycerol. In another embodiment, the extractant mixture comprises N,N-dimethylformamide, glycerol and an auxiliary extractant, wherein the auxiliary extractant is selected from a group consisting of sulfolane, dimethyl sulfoxide, and phenol. The auxiliary extractant is selected in an amount so that the extractive distillation is not affected. In a preferred embodiment, the extractant mixture comprises 0-50% by weight, preferably 0-20% by weight, and more preferably 0-10% by weight of the auxiliary extractant. N,N-dimethylformamide and glycerol are in a total amount of 50-100% by weight, preferably 80-100% by weight, and more preferably 90-100% by weight of the extractant mixture. Preferably, in the above two embodiments, N,N-dimethylformamide and glycerol are in a mass ratio of 0.05-0.5:1, preferably 0.06-0.12:1. The inventors of the present invention surprisingly found that controlling N,N-dimethylformamide and glycerol in a mass ratio within the above range is helpful for obtaining a better separation effect.

In the embodiment where the extractant mixture comprises an auxiliary extractant, the bottom fraction comprises vinyltoluene, glycerol and the auxiliary extractant. Accordingly, in step (3), the bottom fraction may be refined in a refining column for vinyltoluene, to obtain a stream enriched in vinyltoluene at the top of the refining column, and a stream containing glycerol and the auxiliary extractant at the bottom of the refining column.

According to a preferred embodiment of the invention, the extractant mixture is introduced from the upper part of the extractive distillation column, and light cracked C9 aromatic hydrocarbons is introduced from the middle part of the extractive distillation column. Such arrangements may result in better countercurrent contact between the extractant mixture and fractions in the extractive distillation column.

According to a preferred embodiment of the invention, the extractant mixture is fed at a temperature of 10-30°C, or at room temperature (25°C).

According to a preferred embodiment of the invention, the extractant mixture and light cracked C9 aromatic hydrocarbons are introduced into the extractive distillation column in a mass flow ratio of 0.1-1.5, preferably 0.1-0.6, more preferably 0.2-0.4.

According to a preferred embodiment of the invention, the extractive distillation column is provided with a feed line for light cracked C9 aromatic hydrocarbons, a feed line for the extractant mixture, a discharge line for the top fraction of the extractive distillation column, and a discharge line for the bottom fraction of the extractive distillation column; wherein the extractive distillation column is connected to the refining column for C9 saturated aromatic hydrocarbons through the discharge line for the top fraction of the extractive distillation column, and is connected to the refining column for vinyltoluene through the discharge line for the bottom fraction of the extractive distillation column.

In step (2), N,N-dimethylformamide obtained at the top of the refining column may be recycled to the extractive distillation column for reuse. In step (3), the glycerol and optionally the auxiliary extractant obtained at the bottom of the refining column may be recycled to the extraction distillation column for reuse.

The refining column for C9 saturated aromatic hydrocarbons is provided with a discharge line for a top fraction at the top of the refining column and a discharge line for a bottom fraction at the bottom of the refining column; wherein the discharge line for the top fraction is connected to the feed line for the extractant mixture.

The refining column for vinyltoluene is provided with a discharge line for a top fraction at the top of the refining column and a discharge line for a bottom fraction at the bottom of the refining column; wherein the discharge line for the bottom fraction of the refining column for vinyltoluene is connected to the feed line for the extractant mixture.

Preferably, the extractive distillation column is operated under the conditions of: a theoretical plate number of 50-90, a pressure of 5-20 kPa, a column temperature of 250-330°C, and a reflux ratio of 15-25.

Preferably, the refining column for C9 saturated aromatic hydrocarbons is operated under the conditions of: a theoretical plate number of 40-80, a normal pressure, a reflux ratio of 10-25, and a column temperature of 250-265°C.

Preferably, the refining column for vinyltoluene is operated under the conditions of: a theoretical plate number of 50-90, a normal pressure, a reflux ratio of 5-20, and a column temperature of 280-380°C.

In a further aspect, provided in the present disclosure is an extractant mixture for separating vinyltoluene and C9 saturated aromatic hydrocarbons represented by C₉H₁₂ via extractive distillation, wherein the extractant mixture comprises N,N-dimethylformamide and glycerol, which are in a mass ratio of 0.05-0.5:1, preferably 0.06-0.12:1. In one embodiment, the extractant mixture used in the invention consists of N,N-dimethylformamide and glycerol. In another embodiment, the extractant mixture comprises N,N-dimethylformamide, glycerol and an auxiliary extractant, wherein the auxiliary extractant is selected from a group consisting of sulfolane, dimethyl sulfoxide, and phenol. The auxiliary extractant is selected in an amount so that the extractive distillation is not affected. In a preferred embodiment, the extractant mixture comprises 0-50% by weight, preferably 0-20% by weight, and more preferably 0-10% by weight of the auxiliary extractant. N,N-dimethylformamide and glycerol are present in a total amount of 50-100% by weight, preferably 80-100% by weight, and more preferably 90-100% by weight of the extractant mixture.

For example, the present disclosure may include following embodiments:
1. A method for separating 2-methylstyrene and mixed aromatic hydrocarbons in light cracked C9 aromatic hydrocarbons via extractive distillation, comprising steps of:
   (1) feeding light cracked C9 aromatic hydrocarbons as a raw material and an extractant into an extractive distillation column and subjecting them to extractive distillation, to obtain a fraction enriched in the mixed aromatic hydrocarbons at the top of the column and a fraction enriched in 2-methylstyrene at the bottom of the column; wherein the extractant is a composite solution of N,N-dimethylformamide and glycerol;
   (2) feeding the fraction enriched in the mixed aromatic hydrocarbons obtained at the top of the column in step (1) into a refining column for mixed aromatic hydrocarbons and subjecting it to refining, to obtain an extractant at the top of the refining column which is recycled to the extractive distillation column for reuse, and mixed aromatic hydrocarbons with high quality at the bottom of the refining column;
   (3) feeding the fraction enriched in 2-methylstyrene obtained at the bottom of the column in step (1) into a refining column for 2-methylstyrene and subjecting it to refining, to obtain 2-methylstyrene with high purity at the top of the refining column, and an extractant at the bottom of the refining column which is recycled to the extractive distillation column for reuse.
2. The method for separating 2-methylstyrene and mixed aromatic hydrocarbons in light cracked C9 aromatic hydrocarbons via extractive distillation of embodiment 1, wherein light cracked C9 aromatic hydrocarbons is what is obtained after removing dicyclopentadiene and removing heavy components from cracked C9 aromatic hydrocarbons.
3. The method for separating 2-methylstyrene and mixed aromatic hydrocarbons in light cracked C9 aromatic hydrocarbons via extractive distillation of embodiment 2, wherein the heavy components are those having more than ten carbon atoms.
4. The method for separating 2-methylstyrene and mixed aromatic hydrocarbons in light cracked C9 aromatic hydrocarbons via extractive distillation of embodiment 2, wherein light cracked C9 aromatic hydrocarbons are C6-C9 aromatic hydrocarbons.
5. The method for separating 2-methylstyrene and mixed aromatic hydrocarbons in light cracked C9 aromatic hydrocarbons via extractive distillation of embodiment 1, wherein N,N-dimethylformamide and glycerol are in a mass ratio of 0.05-0.5:1, preferably 0.06-0.12:1.
6. The method for separating 2-methylstyrene and mixed aromatic hydrocarbons in light cracked C9 aromatic hydrocarbons via extractive distillation of embodiment 1, wherein the extractant is introduced from the distillation part of the extractive distillation column, and the extractant is fed at a temperature of 10-30°C.
7. The method for separating 2-methylstyrene and mixed aromatic hydrocarbons in light cracked C9 aromatic hydrocarbons via extractive distillation of embodiment 1, wherein the extractant and the raw material are in a mass flow ratio of 0.1-1.5, preferably 0.1-0.6, and more preferably 0.2-0.4.
8. The method for separating 2-methylstyrene and mixed aromatic hydrocarbons in light cracked C9 aromatic hydrocarbons via extractive distillation of embodiment 1, wherein the extractive distillation column is operated under the conditions of: a theoretical plate number of 50-90, a pressure of 5-20 kPa, a column temperature of 300-330°C, and a reflux ratio of 15-25.
9. The method for separating 2-methylstyrene and mixed aromatic hydrocarbons in light cracked C9 aromatic hydrocarbons via extractive distillation of embodiment 1, wherein the refining column for mixed aromatic hydrocarbons is operated under the conditions of: a theoretical plate number of 40-80, a normal pressure, a reflux ratio of 10-25, and a column temperature of 250-265°C.
10. The method for separating 2-methylstyrene and mixed aromatic hydrocarbons in light cracked C9 aromatic hydrocarbons via extractive distillation of embodiment 1, wherein the refining column for 2-methylstyrene is operated under the conditions of: a theoretical plate number of 50-90, a normal pressure, a reflux ratio of 5-20, and a column temperature of 360-380°C.

### Examples

The invention will be further described with reference to examples, without limiting the scope of the invention thereto.

Light cracked C9 aromatic hydrocarbons used in the following examples consisted of (by weight): 5.6% allylbenzene, 3.7% n-propylbenzene, 10.6% 2-ethyltoluene, 2.3% 3-ethyltoluene, 3.7% 4-ethyltoluene, 7.2% 1,3,5-trimethylbenzene, 2.8% 1,2,4-trimethylbenzene, 1.8% 1,2,3-trimethylbenzene, 6.6% isopropylbenzene, 55.1% 2-methylstyrene, and 0.6% dicyclopentadiene.

The examples all followed the process flow as shown in Fig. 1. In particular, light cracked C9 aromatic hydrocarbons (a) were introduced from the middle part of an extractive distillation column (1), and an extractant mixture (b) was introduced from the upper part of the extractive distillation column (1) at room temperature, thereby subjecting them to extractive distillation, to obtain a top fraction and a bottom fraction. The top fraction of the extractive distillation column (1) was fed into a refining column for C9 saturated aromatic hydrocarbons (2) and subjected to refining, to obtain DMF at the top of the refining column and a stream enriched in C9 saturated aromatic hydrocarbons (c) at the bottom of the refining column, wherein DMF obtained at the top of the refining column was recycled to the extractive distillation column (1) for reuse. The bottom fraction of the extractive distillation column (1) was fed into a refining column for vinyltoluene (3) and subjected to refining, to obtain a stream enriched in vinyltoluene (d) at the top of the refining column, and GLY and optionally an auxiliary extractant at the bottom of the refining column, wherein GLY and optionally the auxiliary extractant obtained at the bottom of the refining column were recycled to the extractive distillation column (1) for reuse.

### Method for testing

Tested were the qualities of the stream enriched in vinyltoluene (d) and vinyltoluene therein, and the qualities of the stream enriched in C9 saturated aromatic hydrocarbons (c) and the C9 saturated aromatic hydrocarbons therein, which were obtained in the examples and comparative examples. The corresponding purity and yield were calculated. With respect to light cracked C9 aromatic hydrocarbons used, the C9 saturated aromatic hydrocarbons were n-propylbenzene, 2-ethyltoluene, 3-ethyltoluene, 4-ethyltoluene, 1,3,5-trimethylbenzene, 1,2,4-trimethylbenzene, 1,2,3-trimethylbenzene and isopropylbenzene, and vinyltoluene was 2-methylstyrene.

The purity and yield were calculated using the following formulas, respectively:
the purity of vinyltoluene = the mass of vinyltoluene/the mass of the stream enriched in vinyltoluene (d) × 100%;
the purity of C9 saturated aromatic hydrocarbons = the mass of C9 saturated aromatic hydrocarbons/the mass of the stream enriched in C9 saturated aromatic hydrocarbons (c) × 100%;
the yield of vinyltoluene = the mass of vinyltoluene / the mass of vinyltoluene in light cracked C9 aromatic hydrocarbons × 100%; and
the yield of C9 saturated aromatic hydrocarbons = the mass of C9 saturated aromatic hydrocarbons / the mass of C9 saturated aromatic hydrocarbons in light cracked C9 aromatic hydrocarbons × 100%,
wherein, the mass of vinyltoluene in light cracked C9 aromatic hydrocarbons was the product of the content of vinyltoluene (55.1%) and the input amount of light cracked C9 aromatic hydrocarbons; and
the mass of mixed aromatic hydrocarbons in light cracked C9 aromatic hydrocarbons was the product of the content of C9 saturated aromatic hydrocarbons (3.7% + 10.6% + 2.3% + 3.7% + 7.2% + 2.8% + 1.8% + 6.6%) and the input amount of light cracked C9 aromatic hydrocarbons.

### Example 1

(1) Light cracked C9 aromatic hydrocarbons (a) were introduced from the middle part of an extractive distillation column (1), and an extractant mixture (b) (a mixed solution of N,N-dimethylformamide (DMF) and glycerol (GLY) in a mass ratio of 0.1:1) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.5, and wherein the extractive distillation column (1) was operated under conditions of: a theoretical plate number of 60, a pressure of 15 kPa, a column temperature of 320°C, and a reflux ratio of 20.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 15, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 15, and a column temperature of 377°C. A stream enriched in vinyltoluene (d) was obtained at the top of the refining column, and GLY was obtained at the bottom of the refining column.

Purity and yield of vinyltoluene and C9 saturated aromatic hydrocarbons were calculated as described above. The results were shown in Table 1.

### Example 2

(1) Light cracked C9 aromatic hydrocarbons (a) were introduced from the middle part of an extractive distillation column (1), and an extractant mixture (b) (a mixed solution of N,N-dimethylformamide (DMF) and glycerol (GLY) in a mass ratio of 0.15:1) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.5, and wherein the extractive distillation column (1) was operated under conditions of: a theoretical plate number of 60, a pressure of 10 kPa, a column temperature of 310°C, and a reflux ratio of 15.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 70, a reflux ratio of 15, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 70, a reflux ratio of 10, and a column temperature of 377°C. A stream enriched in vinyltoluene (d) was obtained at the top of the refining column, and GLY was obtained at the bottom of the refining column.

Purity and yield of vinyltoluene and C9 saturated aromatic hydrocarbons were calculated as described above. The results were shown in Table 1.

### Example 3

(1) Light cracked C9 aromatic hydrocarbons (a) were introduced from the middle part of an extractive distillation column (1), and an extractant mixture (b) (a mixed solution of N,N-dimethylformamide (DMF) and glycerol (GLY) in a mass ratio of 0.12:1) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.5, and wherein the extractive distillation column (1) was operated under conditions of: a theoretical plate number of 70, a pressure of 10 kPa, a column temperature of 330°C, and a reflux ratio of 20.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 15, and a column temperature of 259°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 20, and a column temperature of 377°C. A stream enriched in vinyltoluene (d) was obtained at the top of the refining column, and GLY was obtained at the bottom of the refining column.

Purity and yield of vinyltoluene and C9 saturated aromatic hydrocarbons were calculated as described above. The results were shown in Table 1.

### Example 4

(1) Light cracked C9 aromatic hydrocarbons (a) were introduced from the middle part of an extractive distillation column (1), and an extractant mixture (b) (a mixed solution of N,N-dimethylformamide (DMF) and glycerol (GLY) in a mass ratio of 0.35:1) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.3, and wherein the extractive distillation column (1) was operated under conditions of: a theoretical plate number of 80, a pressure of 10 kPa, a column temperature of 300°C, and a reflux ratio of 15.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 20, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 15, and a column temperature of 377°C. A stream enriched in vinyltoluene (d) was obtained at the top of the refining column, and GLY was obtained at the bottom of the refining column.

Purity and yield of vinyltoluene and C9 saturated aromatic hydrocarbons were calculated as described above. The results were shown in Table 1.

### Example 5

(1) Light cracked C9 aromatic hydrocarbons (a) were introduced from the middle part of an extractive distillation column (1), and an extractant mixture (b) (a mixed solution of N,N-dimethylformamide (DMF) and glycerol (GLY) in a mass ratio of 0.08:1) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.3, and wherein the extractive distillation column (1) was operated under conditions of: a theoretical plate number of 60, a pressure of 10 kPa, a column temperature of 320°C, and a reflux ratio of 20.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 20, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 10, and a column temperature of 377°C. A stream enriched in vinyltoluene (d) was obtained at the top of the refining column, and GLY was obtained at the bottom of the refining column.

Purity and yield of vinyltoluene and C9 saturated aromatic hydrocarbons were calculated as described above. The results were shown in Table 1.

### Example 6

(1) Light cracked C9 aromatic hydrocarbons (a) were introduced from the middle part of an extractive distillation column (1), and an extractant mixture (b) (a mixed solution of N,N-dimethylformamide (DMF) and glycerol (GLY) in a mass ratio of 0.24:1) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.3, and wherein the extractive distillation column (1) was operated under conditions of: a theoretical plate number of 60, a pressure of 20 kPa, a column temperature of 310°C, and a reflux ratio of 15.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 70, a reflux ratio of 15, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 15, and a column temperature of 377°C. A stream enriched in vinyltoluene (d) was obtained at the top of the refining column, and GLY was obtained at the bottom of the refining column.

Purity and yield of vinyltoluene and C9 saturated aromatic hydrocarbons were calculated as described above. The results were shown in Table 1.

### Example 7

(1) Light cracked C9 aromatic hydrocarbons (a) were introduced from the middle part of an extractive distillation column (1), and an extractant mixture (b) (a mixed solution of N,N-dimethylformamide (DMF) and glycerol (GLY) in a mass ratio of 0.43:1) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.3, and wherein the extractive distillation column (1) was operated under conditions of: a theoretical plate number of 60, a pressure of 10 kPa, a column temperature of 295°C, and a reflux ratio of 20.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 20, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 10, and a column temperature of 372°C. A stream enriched in vinyltoluene (d) was obtained at the top of the refining column, and GLY was obtained at the bottom of the refining column.

Purity and yield of vinyltoluene and C9 saturated aromatic hydrocarbons were calculated as described above. The results were shown in Table 1.

### Example 8

(1) Light cracked C9 aromatic hydrocarbons (a) were introduced from the middle part of an extractive distillation column (1), and an extractant mixture (b) (a mixed solution of N,N-dimethylformamide (DMF) and glycerol (GLY) in a mass ratio of 0.06:1) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.3, and wherein the extractive distillation column (1) was operated under conditions of: a theoretical plate number of 60, a pressure of 10 kPa, a column temperature of 301°C, and a reflux ratio of 20.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 20, and a column temperature of 258°C. DMF was obtained at the top of the refining column, which was optionally recycled to the extractive distillation column (1) for reuse, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 10, and a column temperature of 374°C. A stream enriched in vinyltoluene (d) was obtained at the top of the refining column, and GLY was obtained at the bottom of the refining column, which was optionally recycled to the extractive distillation column for reuse.

Purity and yield of vinyltoluene and C9 saturated aromatic hydrocarbons were calculated as described above. The results were shown in Table 1.

### Example 9

(1) Light cracked C9 aromatic hydrocarbons (a) were introduced from the middle part of an extractive distillation column (1), and an extractant mixture (b) (a mixed solution of N,N-dimethylformamide (DMF), glycerol (GLY) and dimethyl sulfoxide (as an auxiliary extractant) in a mass ratio of 1:9:90) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.3, and wherein the extractive distillation column (1) was operated under conditions of: a theoretical plate number of 60, a pressure of 10 kPa, a column temperature of 277°C, and a reflux ratio of 20.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 20, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 10, and a column temperature of 290°C. A stream enriched in vinyltoluene (d) was obtained at the top of the refining column, and a mixture of GLY and dimethyl sulfoxide was obtained at the bottom of the refining column.

Purity and yield of vinyltoluene and C9 saturated aromatic hydrocarbons were calculated as described above. The results were shown in Table 1.

### Example 10

(1) Light cracked C9 aromatic hydrocarbons (a) were introduced from the middle part of an extractive distillation column (1), and an extractant mixture (b) (a mixed solution of N,N-dimethylformamide (DMF), glycerol (GLY) and sulfolane in a mass ratio of 1:9:10) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.3, and wherein the extractive distillation column (1) was operated under conditions of: a theoretical plate number of 60, a pressure of 10 kPa, a column temperature of 280°C, and a reflux ratio of 20.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 20, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 10, and a column temperature of 377°C. A stream enriched in vinyltoluene (d) was obtained at the top of the refining column, and a mixture of GLY and sulfolane was obtained at the bottom of the refining column.

Purity and yield of vinyltoluene and C9 saturated aromatic hydrocarbons were calculated as described above. The results were shown in Table 1.

### Example 11

(1) Light cracked C9 aromatic hydrocarbons (a) were introduced from the middle part of an extractive distillation column (1), and an extractant mixture (b) (a mixed solution of N,N-dimethylformamide (DMF), glycerol (GLY) and phenol in a mass ratio of 1:9:10) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.3, and wherein the extractive distillation column (1) was operated under conditions of: a theoretical plate number of 60, a pressure of 10 kPa, a column temperature of 276°C, and a reflux ratio of 20.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 20, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 10, and a column temperature of 300°C. A stream enriched in vinyltoluene (d) was obtained at the top of the refining column, and a mixture of GLY and phenol was obtained at the bottom of the refining column.

Purity and yield of vinyltoluene and C9 saturated aromatic hydrocarbons were calculated as described above. The results were shown in Table 1.

### Example 12

(1) Light cracked C9 aromatic hydrocarbons (a) were introduced from the middle part of an extractive distillation column (1), and an extractant mixture (b) (a mixed solution of N,N-dimethylformamide (DMF), glycerol (GLY) and dimethyl sulfoxide in a mass ratio of 1:9:10) was introduced from the upper part of the extractive distillation column (1) at room temperature, thereby subjecting them to extractive distillation, to obtain a top fraction and a bottom fraction, wherein the extractant mixture (b) and light cracked C9 aromatic hydrocarbons (a) were in a mass flow ratio of 0.3, and wherein the extractive distillation column (1) was operated under conditions of: a theoretical plate number of 60, a pressure of 10 kPa, a column temperature of 276°C, and a reflux ratio of 20.
(2) The top fraction of step (1) was fed into a refining column for C9 saturated aromatic hydrocarbons (2) and subjected to refining, wherein the refining column for C9 saturated aromatic hydrocarbons (2) was operated under conditions of: at normal pressure, a theoretical plate number of 60, a reflux ratio of 20, and a column temperature of 258°C. DMF was obtained at the top of the refining column, and a stream enriched in C9 saturated aromatic hydrocarbons (c) was obtained at the bottom of the refining column.
(3) The bottom fraction of step (1) was fed into a refining column for vinyltoluene (3) and subjected to refining, wherein the refining column for vinyltoluene (3) was operated under conditions of: at normal pressure, a theoretical plate number of 60, a reflux ratio of 10, and a column temperature of 310°C. A stream enriched in vinyltoluene (d) was obtained at the top of the refining column, and a mixture of GLY and dimethyl sulfoxide was obtained at the bottom of the refining column.

Purity and yield of vinyltoluene and C9 saturated aromatic hydrocarbons were calculated as described above. The results were shown in Table 1.

### Comparative example 1

Example 6 was repeated except that only DMF was used as an extractant.

Purity and yield of vinyltoluene and C9 saturated aromatic hydrocarbons were calculated as described above. The results were shown in Table 1.

### Comparative example 2

Example 6 was repeated except that only GLY was used as an extractant.

Purity and yield of vinyltoluene and C9 saturated aromatic hydrocarbons were calculated as described above. The results were shown in Table 1.

### Comparative example 3

Example 6 was repeated except that the extractant mixture was a mixed solution of N,N-dimethylformamide (DMF) and glycerol (GLY) in a mass ratio of 0.67:1.

Purity and yield of vinyltoluene and C9 saturated aromatic hydrocarbons were calculated as described above. The results were shown in Table 1.

**Table 1**

| | C9 saturated aromatic hydrocarbons | | vinyltoluene | |
|---|---|---|---|---|
| | Purity, % | Yield, % | Purity, % | Yield, % |
| Ex. 1 | 69.8 | 82.9 | 92.4 | 90.1 |
| Ex. 2 | 67.9 | 80.6 | 90.7 | 88.4 |
| Ex. 3 | 70.1 | 83.3 | 92.1 | 89.9 |
| Ex. 4 | 69.9 | 81.8 | 90.6 | 88.3 |
| Ex. 5 | 70.3 | 83.6 | 92.6 | 90.3 |
| Ex. 6 | 68.0 | 80.8 | 90.8 | 88.5 |
| Ex. 7 | 67.8 | 83.0 | 92.3 | 90.1 |
| Ex. 8 | 66.5 | 78.3 | 91.7 | 89.5 |
| Ex. 9 | 64.0 | 78.2 | 90.5 | 86.5 |
| Ex. 10 | 68.2 | 80.1 | 90.8 | 88.0 |
| Ex. 11 | 69.0 | 81.0 | 90.5 | 87.2 |
| Ex. 12 | 69.2 | 80.6 | 90.6 | 87.9 |
| CE. 1 | 0 | 0 | 26.9 | 26.3 |
| CE. 2 | 63.3 | 76.7 | 85.9 | 83.8 |
| CE. 3 | 0 | 0 | 41.3 | 40.3 |

As could be seen from Table 1, the method in accordance with the present disclosure effectively separated vinyltoluene and C9 saturated aromatic hydrocarbons, with improved purity and yield.

The present disclosure is not limited to the special details of the above embodiments. Various simple modifications may be made to the solutions of the present disclosure within the technical scope of the present invention. Those simple modifications should also be regarded as being within the protection scope of the present disclosure.

Further, it should be noted that the specific technical features described in the above embodiments can be combined in any suitable way without contradiction. To avoid unnecessary repetition, various possible combinations are not separately described.

In addition, any combinations of various embodiments of the present disclosure may be made as long as they are not away from the concept of the present disclosure. Those combinations should also be regarded as the contents of the present disclosure.

## Claims

1. A method for separating vinyltoluene and C9 saturated aromatic hydrocarbons represented by C₉H₁₂, comprising steps of:
(1) providing a stream containing vinyltoluene and C9 saturated aromatic hydrocarbons;
(2) feeding the stream and an extractant mixture into an extractive distillation column and subjecting them to extractive distillation, to obtain a top fraction and a bottom fraction, wherein the extractant mixture comprises N,N-dimethylformamide and glycerol, the top fraction comprises C9 saturated aromatic hydrocarbons and N,N-dimethylformamide, and the bottom fraction comprises vinyltoluene and glycerol.

2. The method of claim 1, wherein the stream containing vinyltoluene and C9 saturated aromatic hydrocarbons consists of or essentially consists of vinyltoluene and C9 saturated aromatic hydrocarbons; and/or
the extractant mixture comprises N,N-dimethylformamide, glycerol and an auxiliary extractant, wherein the auxiliary extractant is selected from a group consisting of sulfolane, dimethyl sulfoxide and phenol; and/or
N,N-dimethylformamide and glycerol are present in a mass ratio of 0.05-0.5:1, preferably 0.06-0.12:1.

3. The method of claim 2, wherein the extractant mixture comprises 0-50% by weight, preferably 0-20% by weight, and more preferably 0-10% by weight of the auxiliary extractant, and N,N-dimethylformamide and glycerol are present in a total amount of 50-100% by weight, preferably 80-100% by weight, and more preferably 90-100% by weight of the extractant mixture.

4. A method for separating vinyltoluene and C9 saturated aromatic hydrocarbons represented by C₉H₁₂ from light cracked C9 aromatic hydrocarbons, comprising steps of:
(1) feeding light cracked C9 aromatic hydrocarbons and an extractant mixture into an extractive distillation column and subjecting them to extractive distillation, to obtain a top fraction and a bottom fraction, wherein the extractant mixture comprises N,N-dimethylformamide and glycerol, the top fraction comprises C9 saturated aromatic hydrocarbons and N,N-dimethylformamide, and the bottom fraction comprises vinyltoluene and glycerol.

5. The method of claim 4, further comprising steps of:
(2) feeding the top fraction of step (1) into a refining column for C9 saturated aromatic hydrocarbons and subjecting it to refining, to obtain N,N-dimethylformamide at the top of the refining column and a stream enriched in C9 saturated aromatic hydrocarbons at the bottom of the refining column;
(3) feeding the bottom fraction of step (1) into a refining column for vinyltoluene and subjecting it to refining, to obtain a stream enriched in vinyltoluene at the top of the refining column and glycerol at the bottom of the refining column.

6. The method of claim 4, wherein N,N-dimethylformamide and glycerol are in a mass ratio of 0.05-0.5:1, preferably 0.06-0.12:1; and/or
the extractant mixture comprises N,N-dimethylformamide, glycerol and an auxiliary extractant, wherein the auxiliary extractant is selected from a group consisting of sulfolane, dimethyl sulfoxide and phenol.

7. The method of claim 6, wherein the extractant mixture comprises 0-50% by weight, preferably 0-20% by weight, and more preferably 0-10% by weight of the auxiliary extractant, and N,N-dimethylformamide and glycerol are present in a total amount of 50-100% by weight, preferably 80-100% by weight, and more preferably 90-100% by weight of the extractant mixture.

8. The method of claim 4, wherein the extractant mixture is introduced from the upper part of the extractive distillation column, and the extractant mixture is fed at a temperature of 10-30°C; and/or
the extractant mixture and light cracked C9 aromatic hydrocarbons are in a mass flow ratio of 0.1-1.5, preferably 0.1-0.6, more preferably 0.2-0.4; and/or
the extractive distillation column is operated under the conditions of: a theoretical plate number of 50-90, a pressure of 5-20 kPa, a column temperature of 250-330°C, and a reflux ratio of 15-25.

9. The method of claim 5, wherein the refining column for C9 saturated aromatic hydrocarbons is operated under the conditions of: a theoretical plate number of 40-80, a normal pressure, a reflux ratio of 10-25, and a column temperature of 250-265°C; and/or
the refining column for vinyltoluene is operated under the conditions of: a theoretical plate number of 50-90, a normal pressure, a reflux ratio of 5-20, and a column temperature of 280-380°C.

10. The method of claim 5, wherein, in step (2), N,N-dimethylformamide obtained at the top of the refining column is recycled to the extractive distillation column for reuse, and
in step (3), glycerol obtained at the bottom of the refining column is recycled to the extraction distillation column for reuse.

11. An extractant mixture for separating vinyltoluene and C9 saturated aromatic hydrocarbons represented by C₉H₁₂ via extractive distillation, wherein the extractant mixture comprises N,N-dimethylformamide and glycerol which are in a mass ratio of 0.05-0.5:1, preferably 0.06-0.12:1.

12. The extractant mixture of claim 11, wherein the extractant mixture comprises N,N-dimethylformamide, glycerol and an auxiliary extractant, wherein the auxiliary extractant is selected from a group consisting of sulfolane, dimethyl sulfoxide and phenol.

13. The extractant mixture of claim 12, wherein the extractant mixture comprises 0-50% by weight, preferably 0-20% by weight, and more preferably 0-10% by weight of the auxiliary extractant, and N,N-dimethylformamide and glycerol are present in a total amount of 50-100% by weight, preferably 80-100% by weight, and more preferably 90-100% by weight of the extractant mixture.
